# EUROPÄISCHE PATENTANMELDUNG

(11) **EP 4 606 332 A1**
(43) Veröffentlichungstag der Anmeldung: **27.08.2025**
(21) Anmeldenummer: 24159492.8
(22) Anmeldetag: 23.02.2024
(51) Int. Cl.: A61B 18/04, A61B 18/00, A61B 18/16

(54) **PLASMASONDE**

(71) Anmelder: Erbe Elektromedizin GmbH, 72072 Tübingen (DE)
(72) Erfinder: SCHNITZLER, Uwe, 72074 Tübingen (DE)
(74) Vertreter: Rüger Abel Patentanwälte PartGmbB

(57) **Zusammenfassung**

Ein verbessertes Instrument zur Argon-Plasma-Chirurgie weist einen Kunststoffschlauch und einen sich durch diesen hindurch erstreckenden elektrischen Leiter (17) auf, der durch einen Kohlenstofffaserfaden oder einen Kohlenstofffasern enthaltenden Körper gebildet ist. Die Kohlenstofffasern dienen insbesondere der Stromleitung und der thermischen Kühlung des Plasmafußpunkts, der sich an dem Ende des elektrischen Leiters (17) direkt an den Kohlenstofffasern oder einem von diesem kontaktieren kompakten Körper bildet. Der elektrische Leiter (17) ist in dem Lumen (16) des Schlauchs (15) ohne Zentrierung und somit ohne Zwangsberührung zu der inneren Wandung des Schlauchs (15) angeordnet. Dadurch und durch die hohe thermische Leitfähigkeit des Leiters (17) in Längsrichtung ergibt sich eine geringe thermische Belastung des Schlauchs und somit eine hohe Standzeit des Instruments. Außerdem kann das Instrument infolge dieser Konstruktion mittels halogenfreier Kunststoffe bereitgestellt werden, wodurch eine thermische Entsorgung nach Gebrauch des Instruments problemlos ist. Dies gilt insbesondere, wenn das Instrument ganz oder weitgehend metallfrei ausgebildet ist.

## Beschreibung

Die Erfindung betrifft ein Instrument zur Durchführung chirurgischer Eingriffe an einem menschlichen oder tierischen Patienten. Insbesondere dient das erfindungsgemäße Instrument zur Argon-Plasma-Koagulation von lebendem Gewebe.

Aus der EP 3 769 707 A1 ist ein als Argon-Plasma-Sonde ausgebildetes Instrument zur Gewebekoagulation bekannt. Diese Sonde umfasst einen flexiblen Kunststoffschlauch, durch dessen gesamte Länge sich ein Lumen erstreckt. An das proximale Ende des Schlauchs ist ein speisendes Gerät angeschlossen, über das der Schlauch mit Argon versorgt wird, das ihn in Richtung des distalen Endes durchströmt. In dem Schlauch ist ein elektrischer Leiter angeordnet, der ebenfalls an dem proximalen Ende des Instruments an einen Pol eines in dem Gerät enthaltenen Generators angeschlossen und so mit elektrischer hochfrequenter Spannung zur Plasmaerzeugung versorgt wird. Der andere Pol des Generators ist an eine Neutralelektrode angeschlossen. Der elektrische Leiter erstreckt sich durch das Lumen des Schlauchs bis zu einer Elektrode, die durch ein Metallplättchen gebildet ist, das mit sich beiden einander diametral gegenüberliegenden Kanten in dem Schlauch abstützt und so zentriert gehalten ist. Eine in distaler Richtung weisende Spitze der Elektrode dient dazu, im Betrieb eine elektrische Entladung zu speisen und so den vorbeigehenden Argonfluss zur Erzeugung eines Plasmastroms zu ionisieren.

Zur Verminderung der Temperaturbelastung des Schlauchs durch verbesserte Kühlung der Elektrode ist die Elektrode mit einem Überzug versehen, der die Wärmeverteilung und Übertragung an den vorbeistreichenden Gasstrom fördern soll. Dennoch stellt die Elektrode eine erhebliche Wärmebelastung für den Kunststoffschlauch dar, die die Verwendung eines thermisch belastbaren Kunststoffmaterials für den Schlauch erfordert. Solche Kunststoffmaterialen enthalten häufig Chlor- oder Fluorverbindungen, die bei der Entsorgung solcher Instrumente Schwierigkeiten bereiten. Insbesondere können bei der Verbrennung solcher Materialien Stoffe entstehen, die aufgrund ihrer Giftigkeit und/oder Beständigkeit eine erhebliche Umweltbelastung und Gesundheitsgefahr darstellen können.

Aus der US 2021/0145499 A1 ist ein Kaltplasmainstrument mit einer Austrittsdüse bekannt, durch die das Plasma elektrisch nach außen gezogen werden soll. Als Material für die Elektrode werden Aluminium oder auch Graphen in Betracht gezogen.

Weiter ist es aus dem allgemeinen großtechnischen Bereich bekannt, kompakte Elektroden aus Graphit auszubilden. Es wird dazu auf die EP 0 476 572 A2 und die JP 2018-098122 A verwiesen.

Davon ausgehend ist es Aufgabe der Erfindung, ein Instrument mit geringerer Umweltbelastung zu schaffen.

Diese Aufgabe wird mit dem Instrument nach Anspruch 1 gelöst:
Das erfindungsgemäße Instrument ist zur medizinischen Behandlung biologischer Gewebe eingerichtet. Vorzugsweise ist es als flexible Sonde ausgebildet, die durch den Arbeitskanal eines Endoskops in den Körper eines Patienten eingeführt werden kann. Das Instrument kann jedoch auch einen nicht flexiblen steifen Schaft aufweisen und am proximalen Ende mit einem Handgriff versehen sein, um beispielweise als laparoskopisches Instrument eingesetzte zu werden. Weiter kann es, falls gewünscht, auch als Handgriff mit kurzem Schaft für den offenchirurgischen Einsatz ausgebildet sein.

Das Instrument weist einen Schlauch auf, der im Fall des endoskopischen Einsatzes vorzugsweise flexibel ausgebildet ist. Wenn das Instrument als laparoskopisches Instrument ausgebildet ist, kann der Schlauch aber auch als steifer Rohrschaft ausgebildet sein. Dies gilt auch, wenn das Instrument für den offenchirurgischen Einsatz ausgebildet und vorgesehen ist.

Durch den Schlauch verläuft ein Lumen, das sich von seinem proximalen Ende bis zu seinem distalen Ende erstreckt. An seinem proximalen Ende sind Anschlussmittel vorgesehen, um den Schlauch an eine Gasversorgungsquelle anschließen und sein Lumen mit einem Gasstrom speisen zu können. Außerdem ist an dem proximalen Ende ein elektrischer Anschluss vorgesehen, der zur Einspeisung eines elektrischen Stroms in einen sich durch das Lumen bis zu dem distalen Ende des Schlauchs erstreckenden elektrischen Leiters eingerichtet ist. Als Anschlussmittel und als elektrischer Anschluss kann ein kombinierter Stecker dienen, der sowohl einen Gasanschluss-Stift als auch mindestens einen elektrischen Pin aufweist. Der elektrische Anschluss kann aber auch von dem Gas-Anschlussmittel getrennt als gesonderter Stecker ausgebildet sein.

Der in oder an der Auslassöffnungen endende Leiter besteht ganz oder teilweise aus Kohlenstofffasern oder enthält solche. Die Kohlenstofffasern weisen eine hohe Wärmeleitfähigkeit auf und sind deswegen dazu geeignet, an dem Ende des Leiters infolge des sich dort ausbildenden Plasmas eingetragene Wärme in proximaler Richtung von dem distalen Leiterende weg zu leiten und über einen erheblichen Abschnitt seiner Länge zu verteilen, die ein Mehrfaches des Durchmessers des Leiters beträgt. Das an dem Leiter in Gegenrichtung entlangströmende Gas trägt dann effizient zur Kühlung bei. Daraus resultiert eine Temperatur des Leiters, die so niedrig ist, dass sie auch von nicht besonders temperaturstabilen Kunststoffen ertragen wird. Infolgedessen kann der Schlauch aus einem wenig temperaturbeständigen Kunststoff, insbesondere einem halogenfreien Kunststoff, bestehen. Der Kunststoff kann insbesondere chlor- und/oder fluorfrei sein. Somit ist eine einfache umweltgerechte Entsorgung eines verbrauchten Instruments durch Verbrennung möglich. Es entstehen dann bei der Verbrennung keine gefährlichen Stoffe, die auf Dauer beständig und giftig oder auf sonstige Weise umweltschädlich sind.

Dies gilt auch im Hinblick auf den aus Kohlenstofffasern bestehenden oder diese zumindest maßgeblich enthaltenden elektrischen Leiter, der ebenfalls zu CO₂ oxidierbar ist, ohne dass dabei problematische Chemikalien, Metalle oder Metalloxide anfallen würden. Falls gewünscht kann das erfindungsgemäße Instrument frei von Nickel, Chrom, Molybdän, Kobalt, Silber, Kupfer oder sonstigen teuren oder giftigen Metallen, insbesondere Schwermetallen sein.

Der Wärmeleitfähigkeit des Leiters kommt es besonders entgegen, wenn die Kohlenstofffasern vorwiegend in Richtung des Lumens orientiert angeordnet sind. Die Wärmeleitung in dem Leiter erfolgt damit im Wesentlichen in Faserlängsrichtung, d.h. in Richtung der bevorzugten Wärmeleitfähigkeit der Kohlenstofffaser.

Die Kohlenstofffasern können in einen Körper eingebunden sein, der steif ist. Bevorzugt wird aber die den Leiter als Garn, als gezwirntes Garn, als Zwirn, als Kordel oder als Schnur auszubilden. Die Kohlenstoffasern können auch als umsponnenes oder umwickeltes Roving, als geflochtene Schnur oder als miteinander ganz oder lediglich stellenweise verklebte Fasern vorgesehen sein. Der so gebildete Leiter ist flexibel und kann sich somit an jede Biegung des Schlauchs anpassen, ohne sich dieser zu widersetzen.

Das Garn kann ein Multifilgarn sein, dass aus einer Vielzahl von Monofilen besteht. Es enthält dann viele Kohlenstofffasern, die sich jeweils durchgängig über die gesamte Länge des Leiters erstrecken. Dies schließt aber nicht aus, dass einzelne Kohlenstoffasern eine geringere Länge aufweisen.

Es ist aber auch möglich, als Garn ein Stapelfasergarn zu verwenden, das aus Kohlenstofffasern beschränkter Länge besteht, die kürzer sind als die Gesamtlänge des Leiters. Durch innigen seitlichen Kontakt der Fasern untereinander, z.B. infolge eines Spinnvorgangs, wird dennoch ein guter Wärmetransport in Leiterlängsrichtung erzielt.

Der Leiter kann über seine gesamte Länge einen konstanten Querschnitt aufweisen, wobei der Leiter von seinem proximalen Ende bis zu dem distalen Ende Kohlenstofffasern enthalten oder aus solchen bestehen kann. In einem solchen Fall kann das erfindungsgemäße Instrument besonders leicht an eine gewünschte für den konkreten Einsatz vorgesehene Bedarfslänge angepasst werden. Das Instrument wird dann beispielweise in einer Einheitslänge bereitgestellt, die so groß ist wie die maximal nötige Länge (zum Beispiel 3 m). Wird ein Instrument in kürzerer Form benötigt, kann an dem distalen Ende ein Abschnitt abgetrennt werden, wobei das Instrument dann sofort einsatzfähig ist. Auch kann ein Instrument mit verbrauchtem, z.B. thermisch oder durch Gewebeanlagerung geschädigtem Ende durch eine geringe Kürzung, d.h. durch Abschneiden des geschädigten Endes sofort wieder einsatzfähig gemacht werden.

In beiden oben genannten Fällen dient der Kohlenstofffasern enthaltende, oder aus solchen Fasern bestehende Leiter nicht nur zur Stromleitung in Längsrichtung des Instruments. Vielmehr dient sein distales Ende als Elektrode zur Speisung des Plasmas. Weil der Leiter von Argon umspült und somit von Sauerstoff ferngehalten ist, zeigen die Kohlenstofffasern eine hohe Standzeit und keinen störenden Abbrand. Auch ist keine Zentrierung des Leiters in dem Lumen erforderlich, sodass die Wärmebelastung des Schlauchs an seinem distalen Ende im Bereich des Plasmas gering bleibt.

Es wird aber darauf hingewiesen, dass es möglich ist, an dem distalen Ende des aus Kohlenstofffasern bestehenden oder solche Fasern enthaltenden Leiters eine Metallelektrode angebracht sein kann. Dies kann beispielsweise eine blanke Edelstahlelektrode, eine silberbeschichtete Edelstahlelektrode, eine Graphitelektrode, eine aus einem gut wärmeleitenden Material wie Wolframkarbid (Hartmetall) oder Wolfram bestehende Elektrode oder dergleichen sein. Die Elektrode kann nach Art eines Pinsels, eines Stifts mit spitzen oder stumpfen Ende, als Nadel, als Schlinge oder auch in anderer zweckmäßiger Form ausgebildet sein.

Unabhängig von der Materialbeschaffenheit und Form des distalen Endes des Leiters, kann dieser einen proximalen Abschnitt aufweisen, der durch einen metallischen Leiter, beispielsweise einen Aluminiumdraht, einen Stahldraht oder einen Draht aus einem anderen Metall gebildet ist. Die Ausbildung des Drahts aus Aluminium oder Stahl hat den Vorzug, dass er bei der Verbrennung eines verbrauchen Instruments keine giftigen Reststoffe entstehen lässt. Eisen, Eisenoxid, Aluminium und Aluminiumoxid werden als unschädliche Reststoffe angesehen.

Der Leiter ist vorzugsweise mit einer Isolierung versehen, die für das Instrument eine innere Isolierung bildet, während der Schlauch eine äußere Isolierung ist. Vorzugsweise erstreckt sich die Isolierung über die gesamte Länge des Leiters und sichert so die elektrische Durchschlagfestigkeit. Die Isolierung besteht dabei vorzugsweise aus einem Kunststoff aus der Klasse der Polyimide. Auch andere Kunststoffe mit hohem Isolationsvermögen und niedrigem elektrischen Verlustwinkel sind einsetzbar.

Wenn der Leiter zur elektrischen Isolierung mit einem Kunststoffband aus einem geeigneten Material, beispielsweise einem Polyimid bewickelt ist, wird die Flexibilität des Leiters nicht wesentlich beeinträchtigt, sodass das Instrument sehr flexibel bleibt. Es kann deswegen auch in Endoskopen eingesetzt werden, wenn diese eine große Abwinkelung des Instruments verlangen.

Die Bewicklung ist vorzugsweise eine Kreuzwicklung, d.h., während eine Wickellage sich als rechte Schraube um den Leiter windet, ist die darüber oder darunter liegende Lage als linksgängige Schraube gewickelt. Zwar mag dadurch die Flexibilität des Leiters geringfügig vermindert werden, jedoch kommt dies der elektrischen Isolationsfestigkeit zugute. Die Flexibilität des Leiters ist jedoch auch in diesem Fall ausreichend, um eine Abwinkelung des Instruments in einem Endoskop zu ermöglichen.

Der Leiter ist in dem Lumen vorzugsweise radial beweglich gelagert und zwar wiederum vorzugsweise auf seiner gesamten Länge einschließlich seines distalen Endes. Es hat sich dabei gezeigt, dass auf eine präzise Zentrierung des Leiters in dem Lumen verzichtet werden kann. Durch die fehlende Zentrierung entfällt aber auch ein wärmeübertragender Kontakt zwischen dem Leiter und dem Schlauch, was wiederum der thermischen Entlastung des Schlauchs dient. Dieser kann somit aus einem umweltfreundlichen Kunststoff, beispielsweise einem Kunststoff aus der Klasse der Polylactide oder gegebenenfalls auch aus der Klasse der Silikonkunststoffe bestehen.

Auch bedeutet der Wegfall einer Zentriereinrichtung insbesondere an dem distalen Ende des Instruments, dass das Instrument bedarfsweise gekürzt, d.h. einfach abgeschnitten werden kann. Die Schnittfläche des abgeschnittenen Leiters bildet dann die Elektrode.

Der Schlauch kann aus einem Kunststoff aus der Klasse Polylactide oder der Klasse der Silicone bestehen. Vorzugsweise werden die Polymere zur Herstellung des Schlauchs aus nachwachsenden Rohstoffen mit neutralem CO₂ Fußabdruck gewonnen.

Weitere Einzelheiten vorteilhafter Ausführungsformen der Erfindung ergeben sich aus Unteransprüchen, aus der Zeichnung sowie aus der zugehörigen Beschreibung.

In der Zeichnung zeigen:
Figur 1 ein erfindungsgemäßes Instrument angeschlossen an ein speisendes Gerät, in schematisierter Übersichtsdarstellung,
Figur 2 einen nicht maßstäblichen Längsschnitt durch das distale Ende des Instruments nach Figur 1,
Figur 3 eine nicht maßstäbliche Stirnansicht des Instruments nach Figur 1,
Figur 4 und 5 abgewandelte Ausführungsformen des erfindungsgemäßen Instruments in nicht maßstäblicher Längsschnittdarstellung.

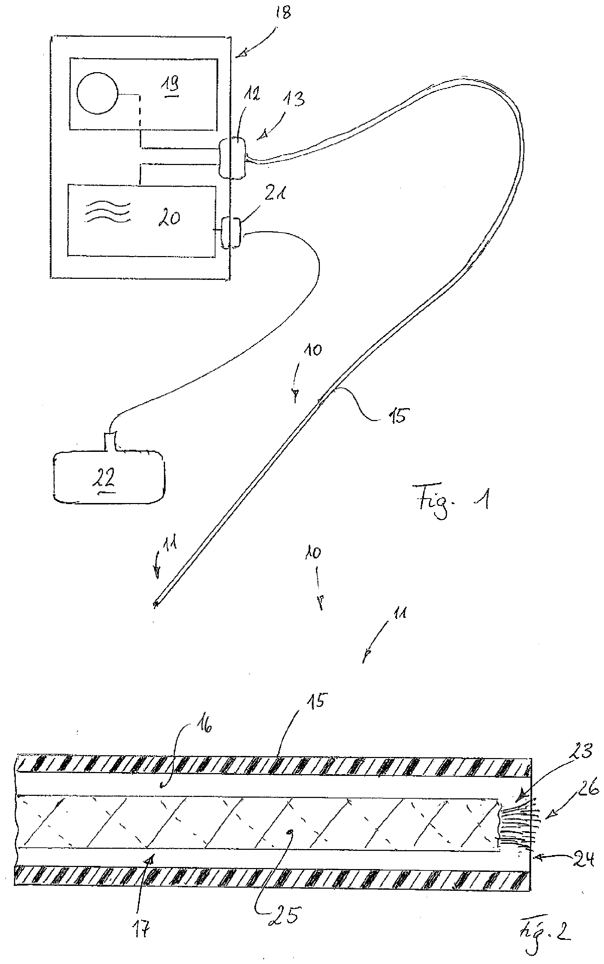

In Figur 1 ist ein als flexible Sonde ausgebildetes Instrument 10 veranschaulicht, das ein durch einen Arbeitskanal eines nicht weiter veranschaulichten Endoskops in einen Patienten einführbares und so an einen Operationsort heranführbares distales Ende 11 aufweist. Weiter weist es ein mit einem Anschlussmittel, beispielsweise einem Stecker 12, versehenes proximales Ende 13 auf. Das Instrument 10 kann aber, anders als dargestellt, auch als laparoskopisches Instrument oder als ein für den offenchirurgischen Einsatz vorgesehenes ausgebildet sein. Ist das Instrument, wie in Figur 1 veranschaulicht, als flexible Sonde ausgebildet, weist es einen sich von dem proximalen Ende 13 bis zu dem distalen Ende 11 erstreckenden Schlauch 15 auf. Ist das Instrument ein laparoskopisches Instrument, kann der Schlauch insbesondere im distalen Bereich als Rohrschaft, d.h. als steifes Rohr ausgebildet sein. Ähnliches gilt für ein für den offenchirurgischen Einsatz vorgesehenes Instrument.

Der Schlauch 15 besteht vorzugsweise aus einem Kunststoff aus der Klasse Polylactide oder der Klasse der Silicone. Durch den Schlauch 15 erstreckt sich von dem proximalen Ende 13 zu dem distalen Ende 11 ein Lumen 16, in dem ein elektrischer Leiter 17 angeordnet ist. Der Leiter 17 erstreckt sich durch die gesamte Länge des Lumens 16.

Der an dem proximalen Ende 13 angeschlossene Stecker 12 dient der Verbindung mit einem speisenden Gerät 18, das eine Quelle 19 für Gas zur Speisung des Lumens 16 enthält. Die Quelle 19 kann zum Beispiel einen Argonspeicher sowie Druckregelarmaturen und Ventile umfassen, um das Lumen 16 kontrolliert mit einem gewünschten Volumenfluss des betreffenden Gases beispielsweise Argon zu versorgen.

Außerdem enthält das Gerät 18 einen elektrischen Generator 20, der einen Ausgangspol aufweist, der über den Stecker 12 mit dem Leiter 17 verbunden ist. Ein anderer Ausgangspool des Generators 20 ist über einen weiteren Stecker 21 und eine elektrische Leitung mit einer Neutralelektrode 22 verbunden, die großflächig mit einem zu behandelnden Patienten zu verbinden ist.

Der Generator 20 ist darauf eingerichtet, eine für die Plasmaerzeugung geeignete hochfrequente Hochspannung zu erzeugen, die mehrere 100 Volt Spitze zu Spitze betragen kann. Die Frequenz der Hochspannung liegt vorzugsweise zwischen 100 kHz und 5 MHz und beträgt typischerweise etwa 350 kHz. Andere Frequenzen sind innerhalb dieses Bereichs möglich. Die Spannung kann ungepulst (CW) ohne Modulation bereitgestellt werden. Der Generator kann auch darauf eingerichtet sein, die Hochspannung in modulierter Form zu liefern, z.B. ein/aus-getastet, d.h. mit einer Rechteckwelle konstanten oder variierenden Puls/Pause-Verhältnisses moduliert. Die Modulationsfrequenz kann unter 100 kHz liegen.

Der elektrische Leiter 17 ist im vorliegenden Ausführungsbeispiel ein aus Kohlenstofffasern 23 bestehender Faden. Die Kohlenstofffasern 23 können miteinander verdrillt, verzwirnt, verflochten oder anderweitig verbunden sein und zusammengehalten sein. Die Kunststofffasern 23 können dabei als Monofile ausgebildet sein. Sie erstrecken sich dann jeweils über die gesamte Länge des Instruments 10 von dem Stecker 12 bis zu dem distalen Ende 11 und der dort vorgesehenen Auslassöffnung 24. Die Kohlenstofffasern 23 können jedoch auch kürzer als die Gesamtlänge des Schlauchs 15 sein, wobei der aus ihnen gebildete Faden dann ein Stapelfasergarn ist. Die einzelnen Fasern können miteinander versponnen sein, um einen Faden zu bilden.

Bei einer bevorzugten Ausführungsform ist der aus den Kohlstofffasern 23 gebildete elektrische Leiter elektrisch isoliert. Die dazu vorgesehene Isolierung 25 kann aus einem Kunststoff mit hohem elektrischem Isolationsvermögen und geringem elektrischen Verlustwinkel bestehen. Beispielsweise kann die Isolierung aus einem Kunststoff aus der Klasse der Polyimide ausgewählt sein.

Unabhängig von der Stoffwahl kann die Isolierung 25 in Form von streifenförmigem Material um den aus parallelliegenden Kohlenstofffasern 23, aus verzwirnten Fasern, aus verdrillten Fasern, verflochtene oder anderweitig miteinander verbundenen Fasern bestehenden Leiter 17 gewickelt sein. Die Isolierung 25 kann einer Schraubenlinie folgend um den Leiter 17 herumgewickelt sein. Bei einer bevorzugten Ausführungsform folgt die Wicklung der Isolierung 25 einer rechten Schraube, wobei eine zweite Lage einer linken Schraube folgt darüber oder darunter liegend angeordnet sein kann. Die doppelte Wicklung ergibt eine Isolierung 25 mit leicht erhöhter Steifigkeit, die aber die Gesamtsteifigkeit des Instruments 10 nicht merklich erhöht. Die durch die Wicklung gebildete Bandagierung des aus Kohlenstofffasern 23 bestehenden Leiters 17, insbesondere die doppelte und in entgegengesetzte Richtung gewickelte Bandagierung, erbringt aber jedenfalls eine zuverlässige elektrische Isolation des Leiters 17. Infolgedessen kann der Schlauch 15 sehr dünnwandig ausgebildet sein, denn die Hauptisolation des elektrischen Leiters 17 wird bereits durch die Isolierung 25 erbracht.

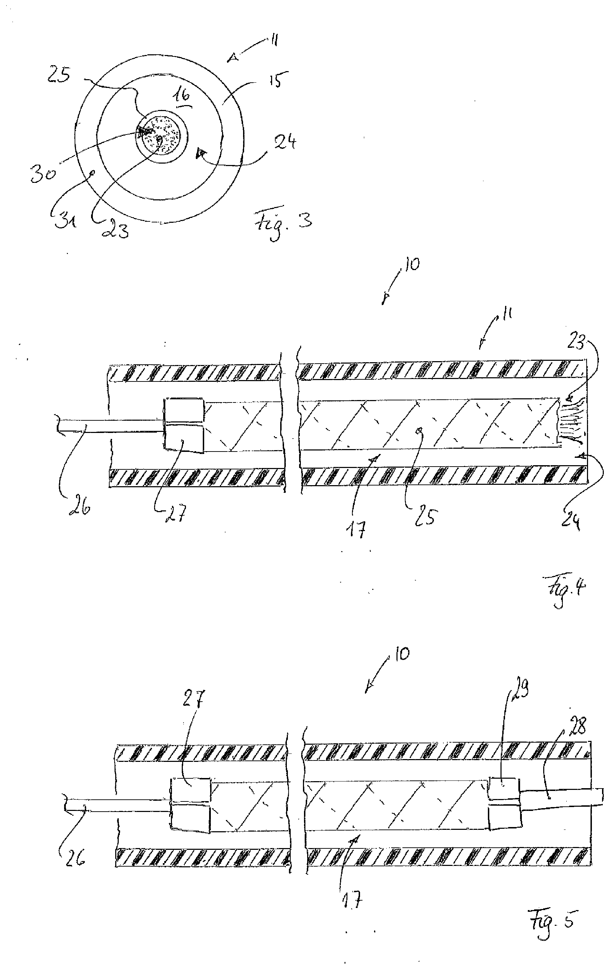

Wie Figur 2 und auch Figur 3 erkennen lässt, liegen die Kohlenstofffasern 23 an dem distalen Ende des Leiters 17 z.B. als Schnittfläche 30 frei. Die Schnittfläche 30 und eine entsprechende End- oder Schnittfläche 31 des Schlauchs 15 können in einer gemeinsamen Ebene oder auch gegeneinander axial versetzt angeordnet sein. Sie können jederzeit durch Einkürzen des Instruments 10 neu erzeugt werden.

Die Kohlenstofffasern 23 bilden an ihren Enden die zur Plasmaerzeugung nötige Elektrode 26. Wie weiter erkennbar ist, ist das Lumen 16 vollständig frei. Es gibt kein Zentrierelement, das den Leiter 17 im Zentrum des Lumens halten würde. Kein mit dem Leiter 17 verbundenes Element liegt unter irgendeiner Vorspannung an der das Lumen 16 umgebenden Fläche des Schlauchs 15 an. Allenfalls können aus dem Ende des Leiters 17 herausschauende Enden der Kohlenstofffasern 23 sich nach Art eines Pinsels aufspreizen und Wandberührung haben. Wegen des vorhandenen elektrischen Widerstands jeder Kohlenstofffaser übernimmt diese jedoch nur anteilig einen von dem elektrischen Widerstand der Kohlenstofffaser abhängigen Bruchteil des Gesamtstroms, sodass die wenigen einzelnen wandberührenden Fasern nur einen geringen Wärmebeitrag liefern.

Das insoweit beschriebene Instrument 10 arbeitet wie folgt:
Zur Inbetriebnahme wird der Stecker 12 mit dem Gerät 18 verbunden und das Instrument 10 direkt oder über einen Arbeitskanal eines Endoskops in den Patienten eingeführt. Zur Behandlung wird die Quelle 19 aktiviert, wodurch ein Argonfluss durch den Kanal 16 in distaler Richtung strömt. Zugleich oder kurz danach wird der Generator 20 aktiviert, sodass nun Hochspannung an den Kohlenstofffasern 23 anliegt. Es bildet sich eine Funkenentladung durch den Argonstrom und somit zu einem Plasmastrom zu dem zu behandelnden Gewebe hin. Der Stromfluss geht dabei von dem Generator über den Leiter 17 durch das Plasma und den Körper des Patienten sowie über die Neutralelektrode 22 zu dem Generator 20 zurück.

Sollte das Instrument 10 im Bereich seiner Auslassöffnung 24 trotz seiner Robustheit thermisch geschädigt worden sein, kann es wieder in gebrauchsfähigen Zustand versetzt werden, indem das Instrument 10 an seinem distalen Ende 11 um einige Millimeter oder Zentimeter gekürzt wird. Dabei werden der Schlauch 15 und der darin liegende Leiter 17 gemeinsam abgeschnitten, um neue Schnittflächen 30, 31 zu erzeugen. Sobald das Instrument 10 dann wieder in Betrieb genommen wird, schmilzt die Isolierung 25 etwas zurück, legt dabei die Kohlenstofffasern 23 frei und das Instrument 10 ist weiterhin uneingeschränkt einsatzfähig.

Die gleiche Prozedur kann vorgenommen werden, wenn das in Standardlänge gelieferte Instrument 10 zu lang ist und beispielsweise um einen oder mehrere Dezimeter gekürzt werden muss. Es kann dann auf die gewünschte Länge abgeschnitten werden, indem von dem distalen Ende 11 die Überschusslänge abgetrennt wird.

Das gebrauchte Instrument 10 kann, wenn es nicht recycelt werden soll oder kann, wie für Krankenhausabfälle üblich verbrannt werden. Bei der beschriebenen Ausführungsform enthält es, außer eventuell im Bereich des Steckers 12, keine metallischen Elemente und besteht letztendlich lediglich aus den Elementen Kohlenstoff und Wasserstoff sowie eventuell noch Stickstoff und Sauerstoff. Es kann damit zu unschädlichen Reststoffen verbrannt werden. Es fallen keine giftigen Abfälle und erst recht keine dauerhaft widerstandsfähigen Gifte an.

Das insoweit grundsätzlich beschriebene Instrument 10 kann, wie zum Beispiel Figur 4 zeigt, auch in abgewandelter Form realisiert werden. Das in Figur 4 veranschaulichte Instrument 10 weist einen Leiter 17 auf, der über einen erheblichen Teil seiner Länge, insbesondere aber im Bereich der Auslassöffnung 24, dem vorbeschriebenen Instrument 10 nach Figur 2 entspricht. Außerhalb seines distalen Abschnitts 11 kann der Leiter 17 aber auch durch einen metallischen Leiter 26 gebildet sein, beispielsweise durch einen Aluminiumdraht, einen Stahldraht, einen Edelstahldraht oder dergleichen. Die Länge des die Kohlenstoffasern 23 enthaltenden Abschnitts des Leiters 17 ist vorzugsweise mindestens so lang, wie der von dem distalen Ende des Instruments 10 zur Kürzung auf ein gewünschtes Minimalmaß oder zur Widerherstellung der Funktion maximal abzuschneidende Abschnitt.

Zur Verbindung mit dem die Kohlenstofffasern 23 enthaltenden Leiter 17 kann eine Crimpverbindung mit einem Crimpring 27 oder auch ein sonstiges geeignetes Verbindungsmittel dienen. Der Crimpring 27 und der elektrische Leiter 17 sind wiederum vorzugsweise mit einer elektrischen Isolierung versehen, die nicht weiter veranschaulicht ist. Auch diese elektrische Isolierung kann wie die Isolierung 25 ausgebildet sein und insbesondere aus einem Polyimid bestehen. Zum Beispiel kann es dabei um einen Polyimidschlauch, eine Polyimidbeschichtung oder auch um eine Bewicklung mit einem oder mehreren Polyimid-Bändern nach dem Vorbild der Isolierung 25 handeln.

Figur 5 veranschaulicht eine weiter abgewandelte Ausführungsform des erfindungsgemäßen Instruments 10. Dieses kann einen Leiter 17 nach Vorbild der Ausführungsform nach Figur 2 aufweisen, der sich über die gesamte Länge des Instruments 10 erstreckt und insgesamt aus Kohlenstofffasern 23 besteht. Der Leiter 17 kann aber auch nach dem Vorbild der Ausführungsform nach Figur 4 ausgebildet sein und in seinem proximalen Abschnitt den metallischen Leiter 26 aufweisen; beides ist möglich. Die Besonderheit der Ausführungsform nach Figur 5 besteht aber darin, dass das distale Ende des Leiters 17 eine kompakte Elektrode 28 trägt. Diese kompakte Elektrode 28 kann eine Nadel, ein Stift oder ein sonstiger Körper aus einem Metall, aus einer elektrisch leitfähigen Keramik oder aus Kohlenstoff, beispielsweise Graphit sein. Die kompakte Elektrode 28 kann wiederum durch einen Crimpring 29 oder eine andere geeignete Befestigung elektrisch und mechanisch mit dem Leiter 17 verbunden sein.

Für alle vorbeschriebenen Ausführungsformen gilt, dass anstelle jedes Crimprings 27, 29 auch andere geeignete Befestigungsmittel, beispielsweise Kunststoffschrumpfelemente oder straffe Wickel aus Draht oder einem geeigneten Bindematerial, zum Beispiel einem Faden aus Kunststoff, Keramikfaser, Glasfaser oder Kohlenstoffaser, Anwendung finden können. Sowohl der metallische Leiter 26 wie auch die kompakte Elektrode 28 können sich jeweils über einen Teil ihrer Länge in den aus Kunststofffasern bestehenden Leiter 17 hinein erstrecken und dadurch eine feste mechanische und elektrische Verbindung zu diesem haben.

Ein verbessertes Instrument 10 zur Argon-Plasma-Chirurgie weist einen Kunststoffschlauch 15 und einen sich durch diesen hindurch erstreckenden elektrischen Leiter 17 auf, der durch einen Faden aus Kohlenstofffasern 23 oder durch einen Kohlenstofffasern 23 enthaltenden flexiblen oder starren Körper gebildet ist. Die Kohlenstofffasern 23 dienen insbesondere der Stromleitung und der thermischen Kühlung des Plasmafußpunkts, der sich an dem Ende des elektrischen Leiters 17 direkt an den Kohlenstofffasern 23 oder einem von diesem kontaktieren kompakten Körper 28 bildet. Der elektrische Leiter 17 ist in dem Lumen 16 des Schlauchs 15 ohne Zentrierung und somit ohne Zwangsberührung zu der inneren Wandung des Schlauchs 15 angeordnet. Dadurch und durch die hohe thermische Leitfähigkeit des Leiters 17 in Längsrichtung, ergibt sich eine geringe thermische Belastung des Schlauchs 15 und somit eine hohe Standzeit des Instruments 10. Außerdem kann das Instrument 10 infolge dieser Konstruktion mittels halogenfreier Kunststoffe bereitgestellt werden, wodurch eine thermische Entsorgung nach Gebrauch des Instruments 10 problemlos ist. Dies gilt insbesondere, wenn das Instrument 10 ganz oder weitgehend metallfrei ausgebildet ist.

### Bezugszeichen:

- 10: Instrument
- 11: distales Ende des Instruments 10
- 12: Stecker
- 13: proximales Ende des Instruments 10
- 15: Schlauch
- 16: Lumen
- 17: Leiter
- 18: Gerät
- 19: Quelle
- 20: Generator
- 21: Stecker
- 22: Neutralelektrode
- 23: Kohlenstoffasern
- 24: Auslassöffnung
- 25: Isolierung
- 26: metallischer Leiter
- 27: Crimpring
- 28: kompakte Elektrode
- 29: Crimpring
- 30, 31: Schnittflächen

## Patentansprüche

1. Instrument (10) zur medizinischer Plasmabehandlung biologischer Gewebe,
mit einem Schlauch (15), der ein proximales Ende (13) und ein distales Ende (11) sowie mindestens ein Lumen (16) aufweist, welches an dem proximalen Ende (13) an eine Gasversorgungsquelle (19) angeschlossen oder anschließbar ist und an dem distalen Ende (11) des Schlauchs (15) an einer Auslassöffnung (24) endet,
mit einem elektrischen Leiter (17), der sich durch die gesamte Länge des Lumens (16) erstreckt und an der Auslassöffnung (24) endet,
**dadurch gekennzeichnet, dass** der Leiter () ganz oder teilweise aus Kohlenstoffasern (23) besteht oder Kohlenstoffasern (23) enthält.

2. Instrument nach Anspruch 1, **dadurch gekennzeichnet, dass** die Kohlenstofffasern (23) vorwiegend in Richtung des Lumens (16) angeordnet sind.

3. Instrument nach einem der vorstehenden Ansprüche, **dadurch gekennzeichnet, dass** der Leiter (17) ein Garn, ein gezwirntes Garn, ein Zwirn, eine Kordel oder eine Schnur ist.

4. Instrument nach Anspruch 3, **dadurch gekennzeichnet, dass** das Garn ein Multifilgarn ist.

5. Instrument nach Anspruch 3, **dadurch gekennzeichnet, dass** das Garn ein Stapelfasergarn ist.

6. Instrument nach einem der vorstehenden Ansprüche, **dadurch gekennzeichnet, dass** der Leiter (17) über seine gesamte Länge einen konstanten Querschnitt aufweist.

7. Instrument nach einem der vorstehenden Ansprüche, **dadurch gekennzeichnet, dass** der Leiter (17) an der Auslassöffnung (24) eine Schnittfläche (30) aufweist.

8. Instrument nach einem der vorstehenden Ansprüche, **dadurch gekennzeichnet, dass** der Leiter (17) mit einer Isolierung (25) versehen ist.

9. Instrument nach Anspruch 8, **dadurch gekennzeichnet, dass** die Isolierung (25) sich über die gesamte Länge des Leiters (17) erstreckend ausgebildet ist.

10. Instrument nach einem der Ansprüche 8 oder 9, **dadurch gekennzeichnet, dass** die Isolierung (25) aus einem Kunststoff aus der Klasse der Polyimide besteht.

11. Instrument nach einem der Ansprüche 8 bis 10, **dadurch gekennzeichnet, dass** der Leiter (17) zur elektrischen Isolierung mit isolierendem Kunststoffband bewickelt ist.

12. Instrument nach einem der vorstehenden Ansprüche, **dadurch gekennzeichnet, dass** der Leiter (17) in dem Lumen (16) radial beweglich gelagert ist.

13. Instrument nach einem der vorstehenden Ansprüche, **dadurch gekennzeichnet, dass** der Leiter (17) in dem distalen Ende (11) des Schlauchs radial frei beweglich liegt.

14. Instrument nach einem der vorstehenden Ansprüche, **dadurch gekennzeichnet, dass** der Schlauch (15) aus einem halogenfreien Kunststoffmaterial besteht.

15. Instrument nach einem der vorstehenden Ansprüche, **dadurch gekennzeichnet, dass** der Schlauch (15) aus einem Kunststoff aus der Klasse der Polylactide oder der Klasse der Silicone besteht.
